# EUROPÄISCHE PATENTANMELDUNG

(11) **EP 2 336 818 A1**
(43) Veröffentlichungstag der Anmeldung: **22.06.2011**
(21) Anmeldenummer: 11002427.0
(22) Anmeldetag: 24.10.2007
(51) Int. Cl.: G02B 23/24, A61B 1/04, A61B 5/00

(54) **Endoskopisches System mit fasergepumpter Fluoreszenzbeleuchtung**

(30) Priorität: 14.11.2006 DE 102006053487
(62) Teilanmeldung aus: 07819267.1
(71) Anmelder: Storz Endoskop Produktions GmbH, 78532 Tuttlingen (DE)
(72) Erfinder: Krattiger, Beat, 8222 Beringen (CH); Klumpp, Martin, 78532 Tuttlingen (DE); Kuster, Manfred, 9443 Widnau (CH); Jäggli, Marcel, 8400 Winterthur (CH); Le Donne-Brechbühl, Sabrina, 8215 Hallau (CH); Hensler, Fritz, 78579 Neuthusen ob Eck (DE)
(74) Vertreter: Stamer, Harald

(57) **Zusammenfassung**

Endoskopisches System (1) mit in einer proximalen Versorgungseinheit (4) angeordneter Anregungsstrahlenquelle (5), mit einer optischen Strahlungs-Übertragungsstrecke in einem Einführungsteil (3) und einem distalseitigen Fluoreszenzumsetzer, wobei als Anregungsstrahlenquelle (5) eine im kurzwelligen sichtbaren Spektralbereich emittierende Laserdiode (6) und als optische Übertragungsstrecke eine Glasfaser (8) vorhanden sind und der Fluoreszenzumsetzer zur Umwandlung in Weißlicht geeignet ist, und bei dem als Fluoreszenzumsetzer ein Fluoreszenzkörper (12, 22) als separates, austauschbares Bauteil der Lichtaustrittsfläche der Glasfaser (8) nachgeordnet ist, zeichnet sich dadurch aus, daß der Fluoreszenzkörper (12, 22) in einem an das Einführungsteil (3) ankoppelbaren Wechselkopf (16) angeordnet ist, der zur Erzeugung eines Beleuchtungs- und/oder Meßstrahlenbündels mit weiteren optischen und wärmeableitenden Bauelementen ausgebildet ist.

## Beschreibung

Die Erfindung betrifft ein endoskopisches System mit den Merkmalen des Oberbegriffs des Anspruchs 1.

Aus JP 2002-148 442 A ist eine Beleuchtungseinrichtung bekannt, bei der das Licht eines Halbleiterlasers in eine optische Glasfaser eingestrahlt wird. Die Glasfaser besteht aus einem lichtleitenden Kern mit hohem Brechungsindex, einem Mantel mit niedrigem Brechungsindex und einer Schutzschicht. In die Schutzschicht sind Fluoreszenzfarbstoffe eingelagert. Der Halbleiterlaser emittiert im Spektralbereich 380 - 460 nm.

Durch Unstetigkeiten und Störstellen im Kern und/oder in der Kern-/Mantel-Grenzschicht wird ein Teil des Lichtes in die Schutzschicht ausgekoppelt. Die Störstellen können von außen an definierter Stelle eingebracht werden. Die Auskopplung kann auch durch Biegen der Glasfaser erreicht werden. Die Fluoreszenzfarbstoffe in der Schutzschicht wandeln blaues Licht des Halbleiterlasers in gelbes Licht um. Ein anderer Teil des ausgekoppelten blauen Lichtes durchdringt die Schutzschicht und addiert sich mit dem gelben Anteil zu Weißlicht. Das Weißlicht wird über die gesamte Länge der Glasfaser abgestrahlt, die mit der Schutzschicht versehen ist und an denen Auskoppelstörstellen vorhanden sind. Die Einrichtung ist im wesentlichen zur Beleuchtung in Anzeigetafeln oder zur Darstellung von Ornamenten vorgesehen.

Eine Weiterentwicklung des Prinzips der Weißlichterzeugung durch additive Farbmischung von blauem Laserlicht und in einem Fluoreszenzumsetzer erzeugten gelben Lichtanteilen ist aus JP 2005-205 195 A bekannt. Das von einer LED oder einer Laserdiode (LD) im blauen Spektralbereich abgestrahlte Licht wird durch eine Kondensoranordnung in eine dünne Multimode-Glasfaser eingespeist. Das andere Ende der Glasfaser ist mit einem Wellenlängen-Umwandlungselement versehen. Dieses besteht aus dem Kern der Glasfaser und einem die Spitze der Glasfaser umhüllenden Fluoreszenzmaterial. Wegen der an der Spitze der Glasfaser konzentrierten Weißlichterzeugung ist die Ausführungsform besonders für endoskopische Anwendungen geeignet. Durch Auswahl der Laser-Emissionswellenlängen und der Komposition des Fluoreszenzmaterials ist eine Vielzahl von Farbabstufungen bei der Fluoreszenzumwandlung und Farbmischung möglich.

Eine optische Einrichtung mit Weißlichterzeugung am distalen Ende der Glasfaser wurde von der Fa. Nichia Corp. Auf der Messe "Laser 2005" in München vorgestellt. Eine blaue Laserdiode speist kurzwelliges, bläuliches Licht mit 405 oder 445 nm Wellenlänge in eine dünne Multimode-Glasfaser ein. An deren Ende befindet sich ein Fluoreszenzumsetzer, der einen Teil des Blaulichts durchläßt und diffus verteilt. Der andere Teil des bläulichen Lichts wird durch den Fluoreszenzfarbstoff in gelbliches Licht umgewandelt und ebenfalls diffus abgestrahlt. Zusammen mit dem direkt durchgelassenen Blaulichtanteil entsteht so durch additive Lichtmischung wiederum ein weißes Licht. Dabei wurde auf eine exakte Abstimmung der Farbstoffe und der Streuung besonderer Wert gelegt, so daß das Licht möglichst neutral wirkt.

Aufgrund der das Faserende umfassenden Beschichtung mit dem Fluoreszenzumsetzer erfolgt die Lichtabstrahlung in einen Winkelbereich von praktisch 360°. Die Glasfaser kann mit dem beschichteten Kopfteil zur Beleuchtung in Hohlräume eingeführt werden, solange die bei der Fluoreszenzumsetzung entstehende Wärme ohne Schaden in den Hohlraum abgestrahlt werden kann.

Aus JP 2005-328 921 A ist ein Adapter für Endoskope bekannt, in den ein Fluoreszenzkörper eingesetzt ist. Der Adapter kann so auf das Distalende des Endoskops aufgesetzt werden, daß der Fluoreszenzkörper der Austrittsfläche einer Beleuchtungsfaser gegenüber liegt. Durch geeignete Formgebung des Fluoreszenzkörpers und Beschichtung seiner Außenfläche kann erreicht werden, daß das Anregungslicht in den Fluoreszenzkörper eintreten kann und das Fluoreszenzlicht in Richtung der Frontfläche des Fluoreszenzkörpers reflektiert wird. Die Frontfläche kann mit einer transparenten Schutzschicht versehen sein.

Neuere Laserlichtquellen werden mit immer größer werdender Leistungsabgabe angeboten. Dies führt zu einer Erhöhung der thermischen Belastung des Fluoreszenzumsetzers, wodurch dessen Lebensdauer herabgesetzt wird. Die thermische Beständigkeit des Fluoreszenzumsetzers kann durch den Übergang von organischen zu anorganischen Fluoreszenzbestandteilen erhöht werden. Das führt dann aber bei höherer Lichtemission zu einer noch höheren Wärmeabstrahlung.

Das Konzept dieser Weißlichterzeugung durch Mischung eines Restes des blauen Anregungslichts mit dem Fluoreszenzlicht ist ähnlich zu dem der ebenfalls bekannten Weißlicht-LEDs. Die Fluoreszenzfarbstoffe sind bei diesen LEDs direkt auf dem blau leuchtenden LED-Chip aufgebracht. Leider haben diese Weißlicht-LEDs den großen Nachteil, daß sie zur Zeit etwa nur den 1- bis 3-fachen Wirkungsgrad von elektrischer Energie (Watt) zu abgestrahltem Licht (Lumen) wie Halogenlampen besitzen. Deshalb entwickeln sie auch sehr viel Abwärme, was sie für endoskopische Anwendungen am Distalende ungeeignet macht. Da die Wärmeableitung am Distalende in der Regel schlecht ist, darf dort durch die Beleuchtung keine große Hitze erzeugt werden, da diese ein Verletzungsrisiko darstellt. Dies ist insbesondere auch bei Videoendoskopen wichtig, da deren distale temperaturempfindliche Kameras selber schon eine gewisse Abwärme erzeugen.

Der Erfindung lag die Aufgabe zugrunde, das Prinzip der bekannten Weißlichterzeugung in endoskopischen Systemen für Beleuchtungs-und Meßstrahlenbündel mit im wesentlichen vorwärts oder gezielt seitwärts gerichteter Lichtabstrahlung nutzbar zu machen und eine Wärmebelastung des Fluoreszenzumsetzers, des Untersuchungsobjekts und/oder der in der Nähe der distalseitigen Beleuchtungsoptik angebrachten endoskopischen Beobachtungssysteme zu vermeiden.

Diese Aufgabe wird bei einem endoskopischen System der eingangs genannten Art erfindungsgemäß durch die kennzeichnenden Merkmale des Anspruchs 1 gelöst. Vorteilhafte Weiterbildungen ergeben sich aus den Merkmalen der Unteransprüche.

Die Anordnung eines von der Glasfaser losgelösten, separaten und damit austauschbaren Fluoreszenzkörpers eröffnet vielfältige Möglichkeiten der geometrischen Formgebung zur Anpassung an die spezifischen Anforderungen eines Endoskops. Aber auch die optischen Eigenschaften des Fluoreszenzkörpers können durch Materialwahl und Materialkomposition vielfältig variiert werden. Außerdem wird die Austauschbarkeit und Montage von Systemeinheiten wesentlich erleichtert.

Neben der Lichtquelle zur Weißlichtabstrahlung kommt in der Endoskopie der Miniaturisierung von Lichtreflektoren und der Strahlformungsoptik eine besondere Bedeutung zu. Wird eine effiziente Strahlformung benötigt, muß der Fluoreszenzkörper aus optischgeometrischen Überlegungen möglichst klein sein gegenüber dem Reflektor oder der Strahlformungsoptik. Diese Miniaturisierung erhöht jedoch zwangsläufig die Wärmekonzentration und den zerstörerischen Temperaturgradienten. Aus diesen Gründen ist die Reduktion der thermischen Widerstände im und um den Fluoreszenzkörper wichtig. In den Unteransprüchen werden Konzepte genannt, wie dies bei miniaturisierten Fluoreszenzkörpern erreicht werden kann.

Die Bezeichnung "Fluoreszenzkörper" soll auch seine Eigenschaft als Streukörper zur Streuung des durchgelassenen Anregungslichtes einschließen. Die Streuung wird durch die im Volumen des Fluoreszenzkörpers eingelagerten Streuzentren und durch Struktureffekte an der Oberfläche bewirkt. Dabei können die Streuzentren gleichzeitig auch die Fluorophore sein. Die Streuzentren können aufgrund ihrer Dimensionierung selektiv die kurzen Wellenlängen bevorzugt streuen.

Ausführungsbeispiele des erfindungsgemäßen Systems sind in der Zeichnung schematisch dargestellt und werden anhand der Figuren näher beschrieben. Dabei zeigen:
- Fig.1: ein endoskopisches System mit Beleuchtungskörper,
- Fig.2: einen Wechselkopf mit quasi punktförmigem Fluoreszenzkörper,
- Fig.3: den Wechselkopf nach Fig.2 mit fokussiertem Anregungsstrahlenbündel und

- Fig.4: den Wechselkopf nach Fig.2 mit kollimiertem Anregungsstrahlenbündel.
- Fig.5a: einen größeren Fluoreszenzkörper in einem Wechselkopf mit seitwärts gerichteter Beleuchtung und Beobachtung,
- Fig.5b: dieselbe Anordnung mit vorwärts gerichteter Beleuchtung und Beobachtung,
- Fig.6: den Wechselkopf nach Fig.5a zusätzlich mit parallelen Meßstrahlenbündeln,
- Fig.7a: den Wechselkopf nach Fig.5a zusätzlich mit Meßmustererzeugung und Fig.7b dieselbe Anordnung zusätzlich mit Videokamera und elektrischen Kontakten.

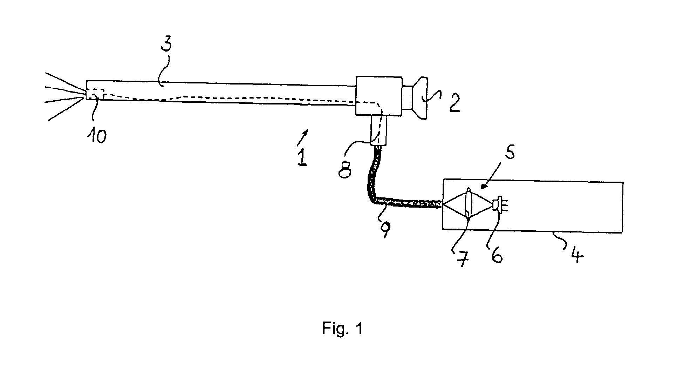

Fig.1 zeigt schematisch ein endoskopisches System 1 mit Okular 2 und Einführungsteil 3. Das Einführungsteil 3 kann als starres Rohr oder flexibel ausgebildet sein. Nach oder anstelle des Okulars mit optischer Übertragung des beobachteten Bildes kann auch eine Videokamera mit Darstellung des beobachteten Bildes auf einem Monitor vorgesehen sein. In einer Versorgungseinheit 4 ist eine Anregungsstrahlenquelle 5 angeordnet, die eine Laserdiode 6 und eine Kopplungsoptik 7 zur Einspeisung des Anregungslichtes in eine Glasfaser 8 enthält. Selbstverständlich ist es auch möglich, weitere Laserdioden mit Emission zusätzlicher Wellenlängen vorzusehen, deren Strahlung ebenfalls in die Glasfaser 8 oder in zusätzliche Glasfasern eingespeist werden kann. Damit können z.B. spektrale Schwächen des Weißlichts ausgeglichen werden. Die Laserdioden können batteriebetrieben sein oder über ein Netzteil mit Energie versorgt werden.

Zur Verbindung der Versorgungseinheit 4 mit dem endoskopischen System 1 ist ein Lichtleiterkabel 9 vorgesehen, das über spezielle oder handelsübliche Steckverbinder am Endoskop und der Versorgungseinheit 4 angeschlossen wird. Die Steckverbinder können insbesondere autoklavierbar und lasergeschützt ausgeführt sein. Durch das Einführungsteil 3 hindurch wird die Glasfaser 8 in üblicher Weise lose oder in einem separaten Beleuchtungskanal oder in einer Schutzhülle zum distalen Ende geführt. Am distalen Ende ist ein Beleuchtungskörper 10 angeordnet, in dem die Umwandlung in Weißlicht und die Strahlformung zur Beleuchtung des Objektraumes oder zu Projektion einer Meßstrahlung erfolgen. Der Beleuchtungskörper 10 ist funktional austauschbar und in einen austauschbaren Wechselkopf am distalen Ende des Einführungsteils 3 integriert. Die Abbildungsoptik ist hier nicht weiter dargestellt.

Das Konzept der Quasi-Punktlichtquelle kann mit einem am distalen Ende des Einführungsrohres 3 angekoppelten Wechselkopf 16 realisiert werden.

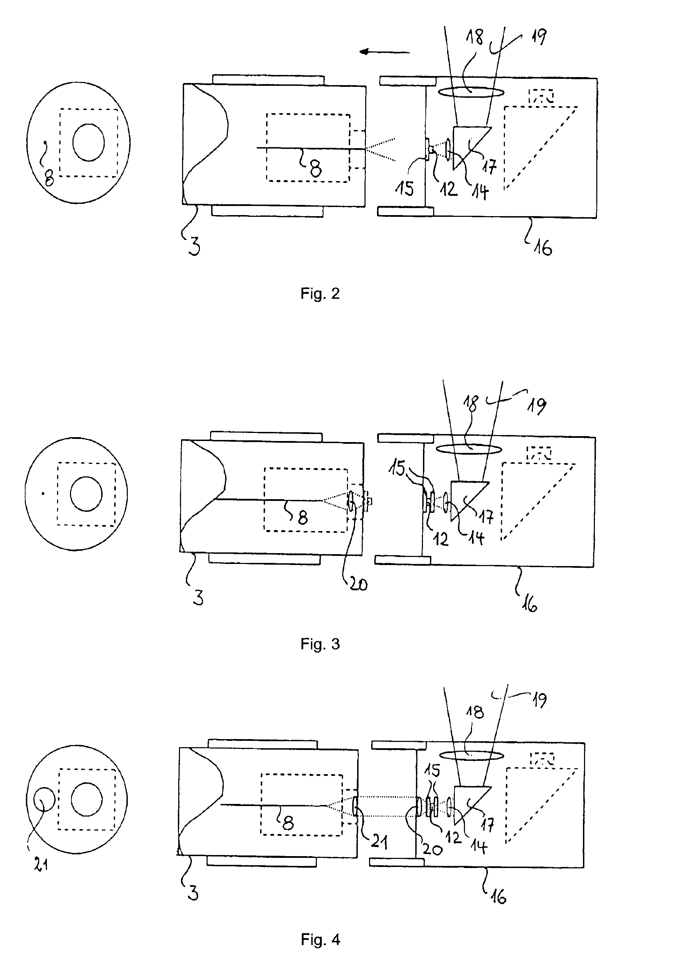

Fig. 2 zeigt eine Ausführungsform, bei der ein kleiner, quasi punktförmiger Fluoreszenzkörper 12 auf einem gut wärmeleitenden Fenster 15, z.B. einer transparenten Keramik-, Saphir- oder Diamantscheibe, angeordnet ist. Der Wechselkopf 16 wird in Pfeilrichtung auf das distale Ende des Einführungsteils 3 geschoben, so daß die Lichtaustrittsfläche der Glasfaser 8 unmittelbar dem Fluoreszenzkörper 12 gegenüber liegt. Diese Anordnung erfordert hohe Positioniergenauigkeiten. Die Bauelemente Fenster 15, Fluoreszenzkörper 12 und Optik 14 können auch in einem Beleuchtungskörper 10 der bereits beschriebenen Art zusammengefaßt und als Einheit in den Wechselkopf 16 eingesetzt sein.

Der Optik 14 sind ein Umlenkprisma 17 und ein Beleuchtungs-Objektiv 18 nachgeordnet, mit denen ein um 90° umgelenkter

Beleuchtungsstrahlenkegel 19 erzeugt wird. Gestrichelt dargestellt sind übliche Bauelemente zur Videoaufnahme des beleuchteten Objekts.

Bei dem in Fig. 3 dargestellten Ausführungsbeispiel ist am distalen Ende des Einführungsteils 3 der Lichtaustrittsfläche der Glasfaser 8 eine Abbildungslinse 20 vorgeschaltet,die das austretende Anregungsstrahlenbündel bei aufgeschobenem Wechselkopf in den Fluoreszenzkörper 12 fokussiert. Der Fluoreszenzkörper 12 ist hier zwischen zwei Wärme ableitenden Fernstern / Scheiben 15 gelagert. Der Fokus der Abbildungslinse 20 ist so eingestellt, daß das Anregungslicht unter Berücksichtigung der Dicke der Scheibe 15 korrekt in den Fluoreszenzkörper 12 hinein fokussiert wird.

Vorteilhaft bei nicht exakt definierter Position des Wechselkopfes 16 ist die parallele Strahlenführung durch die Schnittstelle zwischen Einführungsteil 3 und Wechselkopf 16, wie sie in Fig. 4 dargestellt ist. Am distalen Ende des Einführungsteils 3 ist eine Kollimationslinse 21 angeordnet, die das aus der Lichtaustrittsfläche der Glasfaser 8 austretende Anregungsstrahlenbündel nach unendlich abbildet. In diesem Fall muß das Anregungslicht mit einer im Wechselkopf 16 angeordneten Abbildungslinse 20 auf den Fluoreszenzkörper 12 fokussiert werden. Die Variante ist zwar aufwändiger, sie ermöglicht aber größere Toleranzen bei der Befestigung des Wechselkopfes 16. Mit der kollimierten Strahlführung sind die größten konstruktiven Möglichkeiten offen, da die Weißlicht-Erzeugung an beliebiger Stelle im Wechselkopf 16 vorgesehen werden kann.

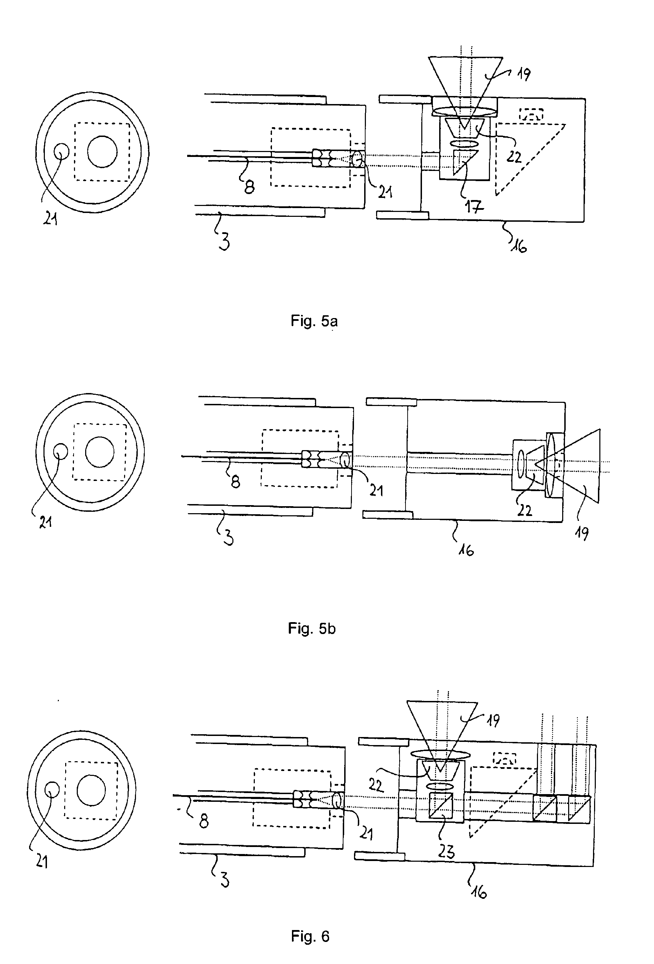

In Fig. 5a ist ein größerer Fluoreszenzkörper 22 dem Umlenkprisma 17 nachgeordnet. In den Fluoreszenzkörper 22 wird daher das kollimierte Anregungsstrahlenbündel eingestrahlt. Wegen der über den Strahlenbündelquerschnitt verteilten Strahlungsdichte wird die Leistungsdichte im Fluoreszenzkörper 22 verringert. Die Reduktion der maximalen Strahlungsdichte verringert vorteilhafterweise das Ausbleichen, die Alterung und die Erwärmung des Fluoreszenzkörpers 22. Bei ausreichender Intensität des Anregungsstrahlenbündels kann ein Teil des Anregungslichtes auch noch direkt durch Fluoreszenzkörper 22 hindurchtreten, wie durch die gepunktete Weiterführung des kollimierten Anregungsstrahlenbündels durch den Beleuchtungsstrahlenkegel 19 hindurch angedeutet ist. Innerhalb des weißen Beleuchtungsstrahlenkegels 19 erscheint dann auf dem beobachteten Objekt ein z.B. blauer Fleck, der als Markierung verwendet werden kann. Die Streueigenschaften des Fluoreszenzkörpers 22 müssen dazu entsprechend angepaßt werden.

Fig. 5b zeigt die gleiche Anordnung, jedoch mit vorwärts gerichteter Beleuchtung und Beobachtung.

Bei dem Ausführungsbeispiel nach Fig. 6 wird das kollimierte Anregungsstrahlenbündel durch einen Strahlenteiler 23 in zwei Strahlenbündel aufgespalten. Der an der Strahlenteilerfläche reflektierte Teil wird zur Umwandlung in Weißlicht verwendet. Aus dem durchgelassenen Teil werden in an sich bekannter Weise über nicht weiter bezeichnete optische Elemente zwei parallele Meßstrahlenbündel erzeugt, die im Bild einen Vergleichsmaßstab zur Bildvermessung bilden. Der am Strahlenteiler 23 durchgelassene Teil des Anregungsstrahlenbündels kann auch zur Anregung eines weiteren Fluoreszenzkörpers verwendet werden. Durch mehrere individuell angeregte Fluoreszenzkörper wird eine schattenfreie Ausleuchtung ermöglicht, die Ausfallsicherheit des Systems verbessert oder es können unterschiedliche Farbspektren oder Abstrahlrichtungen eingestellt werden.

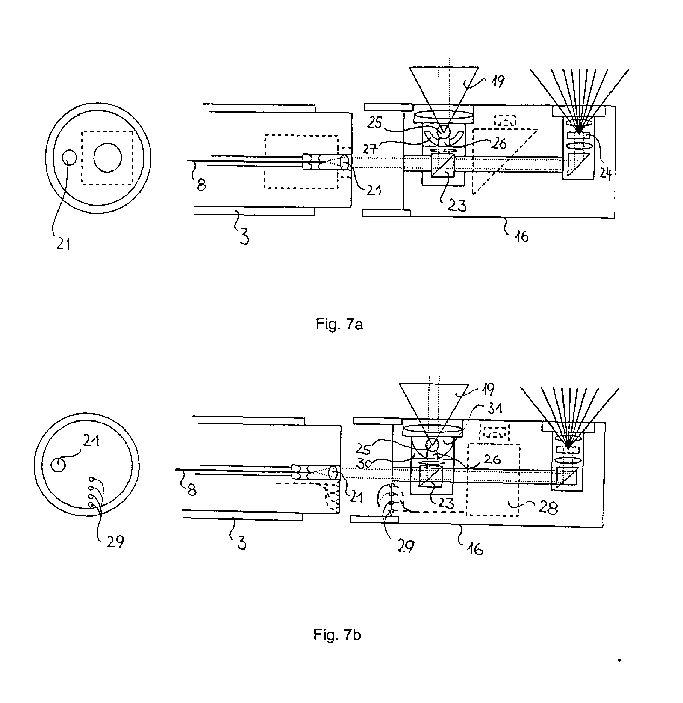

Bei dem Ausführungsbeispiel nach Fig. 7a erfolgt ebenfalls eine Teilung des kollimierten Anregungsstrahlenbündels. Der am Strahlenteiler 23 durchgelassene Teil wird über ein diffraktives optisches Element 24 in eine Vielzahl von Strahlenbündeln zur Erzeugung eines Meßmusters aufgespalten. Der Fluoreszenzkörper 12 ist in diesem Ausführungsbeispiel als Kugel 25 dargestellt, die von einem transparenten, Wärme leitenden Sockel 26 gehalten wird. Der Sockel 26 und die Kugel 25 werden von einem Reflektor 27 umfaßt. Die Kugelform gewährleistet eine gleichförmige Abstrahlung. Die Abführung der Wärme ist wegen der geringen Kontaktfläche am Sockel 26 jedoch ungünstig.

Bei dem in Fig. 7b dargestellten Ausführungsbeispiel sind dieselben Beleuchtungselemente vorgesehen wie in Fig. 7a. Zur Beobachtung des beleuchteten Objektbereichs ist hier jedoch eine Videokamera 28 in den Wechselkopf 16 integriert, die über Kontakte 29 elektrisch mit dem distalen Ende des Einführungsteiles 3 verbunden wird. Der kugelförmige Fluoreszenzkörper 25 ist hier in einen Reflektorkörper 30 eingesetzt, dessen z.B. parabolische innere Reflektorfläche verspiegelt ist. Der Reflektorkörper 30 kann um den kugelförmigen Fluoreszenzkörper 25 herum mit einem transparenten Wärmeleiter 31 ausgefüllt sein.

Bei der Beschreibung der Ausführungsbeispiele wurde zunächst von der Übertragung einer zur Fluoreszenz anregenden Lichtwellenlänge durch die Glasfaser ausgegangen. Es ist jedoch auch möglich, in die Glasfaser das Licht von mehr als einer Laserdiode mit unterschiedlichen Lichtwellenlängen einzuspeisen. Im Wechselkopf 16 muß dann im Strahlenteiler 23 die Strahlenteilerfläche mit einer dichroitischen Beschichtung versehen werden, die für die von der Anregungswellenlänge abweichenden Wellenlängen der Strahlung durchlässig ist. Dadurch kann eine für die Meßstrahlung günstigere Farbe, z.B. rot oder grün, für eine bessere Erkennbarkeit eingesetzt werden.

Die Vorteile der fasergepumpten Fluoreszenzbeleuchtung können wie folgt zusammengefaßt werden:
- Da zur Übertragung des Anregungslichtes im Prinzip nur eine einzige Glasfaser genügt, wird eine bessere Flexibilität des Einführungsteiles gegenüber Verbiegungen bei der Ablenkung des Distalendes erreicht. Die geringere Rückstellkraft einer Einzelfaser gegenüber den herkömmlichen Faserbündeln hat eine Verbesserung der Mechanik zur Folge, da die Einzelfaser viel biegsamer als ein Faserbündel ist. Durch die geringere Rückstellkraft wird der mechanische Ablenkungsvorgang am Distalende präziser.
- Aufgrund des großen Durchmessers herkömmlicher Lichtleiterfaserbündel entstehen beim Biegen jeweils an den Innen-und Außenfasern Scherkräfte, die die Fasern ausreißen oder knicken können. Bei einer Einzelfaser entstehen keine Innen-Außen-Zugkräfte.
- Der Einzelfaserdurchmesser beträgt mit Schutzmantel nur ca. 80-900 Mikrometer. In einem konventionell beleuchteten Videoendoskop beträgt demgegenüber der Kaltlicht-Bündeldurchmesser zwischen ca. 1 bis 3 mm. Ein Endoskop mit Einzelfaserübertragung des Anregungslichtes kann daher insgesamt mit einem wesentlich geringeren Querschnitt konstruiert werden.
- Wenn eine Faser nicht ausreicht, können ohne wesentliche Querschnittserhöhung mehrere Fasern auf einen gemeinsamen Fluoreszenzkörper einstrahlen oder sie können jeweils einen eigenen Fluoreszenzkörper bestrahlen. Somit kann in einfacher Weise die Lichtleistung hochskaliert werden.
- Wenn die Fluoreszenzquelle klein ist gegenüber der Strahlformungsoptik kann die Ausleuchtung dem Gesichtsfeld optimal angepaßt werden.
- Durch die Wahl des oder der Fluoreszenzfarbstoffe im Fluoreszenzkörper und/oder durch die Wahl des Anregungslichtes ist das Farbspektrum anpaßbar. So kann z.B. mit Anregung in UV und blau in der gleichen Faser das Spektrum an die zur Farbwiedergabe optimale Schwarzkörperstrahlung angepaßt werden. Es kann auch Licht zu Zwecke der Streuung ohne Nutzung des Fluoreszenzeffektes eingestrahlt werden. Dazu können verschiedene Lichtquellen z.B. mit einem Faserkoppler in eine Einzelfaser eingespiesen werden. Durch Austauschen eines Wechselkopfes kann ebenfalls ein Spektrumswechsel erfolgen. Bei der Wahl des Anregungslichtes kann das Farbspektrum sogar während des endoskopischen Betrachtens geändert werden, was z.B. vorteilhaft ist bei Untersuchungen auf Farbveränderungen des Untersuchungsobjekts.
- Ist der Fluoreszenzkörper durch Alterung ausgeblichen, gibt er nicht mehr seine maximale Helligkeit ab. Dann ist er z.B. via Austausch eines Wechselkopfes auswechselbar. Es ist auch möglich, nur den Beleuchtungskörper oder nur den Fluoreszenzkörper auszutauschen, wodurch dann ein Maximum an Wiederverwendung der Teile möglich wird. Dies spart Gebrauchskosten gegenüber fest eingebauten Fluoreszenzsystemen.
- Weil die Laserdiode in der Versorgungseinheit als Receptacle ausgestaltet ist, kann sie bei Defekt mit dem Receptacle jederzeit ausgetauscht werden. Falls in Zukunft Laserdioden mit größerer Lichtleistung erhältlich sind, kann das endoskopische System jederzeit auf einfache Art aufgerüstet werden, womit die Lichtleistung am Distalende erhöht werden kann. Erfordern dann die höhere Leistung oder eine veränderte Wellenlänge eine Anpassung des Fluoreszenzkörpers, so ist das wegen der erfindungsgemäßen Austauschbarkeit möglich.
- Durch die steckbare Verbindung der Übertragungsfaser zur Laserdiode und durch die Positionierung des Fluoreszenzkörpers als separates Bauteil ist die Faser jederzeit austauschbar. Dies ist ein wesentlicher Servicevorteil, da die Faser im Betrieb brechen oder reißen kann.
- Bei Verwendung von energieeffizienten Laserdioden zur Speisung der Faser ist ein Batteriebetrieb möglich. Dadurch wird ein mobiler Einsatz des Systems erleichtert.
- Durch den Einsatz von größeren Lasern ist die Übertragung von Lichtleistungen bis zu einigen Watt ans Distalende möglich. Die distal abgegebene Lichtmenge wird dann nur durch den Fluoreszenzkörper und dessen thermische Einbindung begrenzt. Durch die Einstrahlung hoher Intensitäten ist es auch möglich, nichtlineare Effekte auszunutzen.
- In der Endoskopie stört oft der Effekt, daß bei langen flexiblen Endoskopen, z.B. ab 5 m, das Beleuchtungslicht mit zunehmender Länge zunehmend gelblich wird. Dies rührt von den stärkeren Lichtverlusten der kurzwelligen Spektralanteile in den Lichtleitern her. Wenn mit einem Laser angeregt wird, ist dagegen nur eine Wellenlänge vorhanden. Dadurch ist keine Veränderung des Anregungsspektrums mit der Länge möglich, so daß nach der Umwandlung auch das ausgestrahlte Licht seine Farbe weitgehend unabhängig von der Länge des Endoskops beibehält. Minimale Farbänderungen wegen nicht linearer Umwandlung lassen sich durch Leistungsanpassung bei Bedarf beheben.
- Die Fasern für Laserübertragung weisen bei Einstrahlung des Laserlichts mit kleiner numerischer Apertur eine geringere Dämpfung auf als die üblicherweise verwendeten Weißlichtfasern, bei denen die Einstrahlung konventioneller Beleuchtung mit hoher numerischer Apertur erfolgt. Mit dem neuen Beleuchtungssystem sind daher wesentlich längere Endoskope möglich.
- Die konventionellen Lichtquellen, wie z.B. Halogenlampen oder Gasentladungslampen, sind technisch an den physikalischen Grenzen angelangt. Bei den Laserdioden oder den Fluoreszenzkörpern ist jedoch zu erwarten, daß ihre Leistungen noch erhöht werden können. Die Technik des neuen Beleuchtungssystems wird daher von der Weiterentwicklung der Komponenten profitieren.
- Die Intensität des Fluoreszenzlichts ist dimmbar, ohne daß sich die Farbe wesentlich ändert. Mechanische Teile, wie Blenden oder Absorber sind für eine Abschwächung nicht erforderlich. Eine einfache Reduktion des Anregungslichts reduziert entsprechend die Ausstrahlung des umgewandelten Lichts. Völlig farbneutrale Dimmung ist hingegen durch einfache Pulsweiten-Modulation möglich.
- Bei Laserdioden kann durch Modulation des Laserstroms die Intensität des Anregungslichts schnell und mit wenig Aufwand verändert werden. Durch Unterbrechung oder Variation des Anregungslichts kann nahezu sofort z.B. das umgewandelte Licht ausgeschaltet werden. Es muß lediglich noch das extrem kurze Nachleuchten des Fluoreszenzkörpers abgewartet werden. Diese schnelle Modulierbarkeit ist bei Topografie-Meßaufgaben vorteilhaft, die nur kurzzeitig eine spezifische Meßbeleuchtung ohne Weißlichtbeleuchtung erfordern.
- Die Montage des Endoskops wird vereinfacht, da keine Faserbäume eingezogen werden müssen.
- Die Reparaturmöglichkeiten des Endoskops werden verbessert, da der Austausch einzelner Fasern einfacher ist als der Austausch eines Faserbündels.
- Es entfallen die Probleme bei der Abdichtung der porösen Enden der Faserbündel gegen Eindringen von Flüssigkeiten.
- Es ist eine Mehrwellenlängen-Anregung mit UV und blau in der gleichen Faser möglich, um z.B. das Spektrum an die Schwarzkörperstrahlung besser anzugleichen. Die Einspeisung in dieselbe Faser kann mit einem Faserkoppler nahezu verlustfrei erfolgen.

### Bezugszeichenliste

- 1: Endoskopisches System
- 2: Okular
- 3: Einführungsteil
- 4: Versorgungseinheit
- 5: Anregungsstrahlenquelle
- 6: Laserdiode
- 7: Kopplungsoptik
- 8: Glasfaser
- 9: Lichtleiterkabel
- 10: Beleuchtungskörper
- 12: Fluoreszenzkörper
- 14: optisches Element
- 15: Wärme ableitende Scheibe / Fenster
- 16: Wechselkopf
- 17: Umlenkprisma
- 18: Beleuchtungs-Objektiv
- 19: Beleuchtungsstrahlenkegel
- 20: Abbildungslinse
- 21: Kollimationsoptik
- 22: größerer Fluoreszenzkörper
- 23: Strahlenteiler
- 24: Diffraktives Optisches Element
- 25: kugelförmiger Fluoreszenzkörper

- 26: Wärme leitender Sockel
- 27: Reflektor
- 28: Videokamera
- 29: elektrische Kontakte
- 30: Reflektorkörper
- 31: transparenter Wärmeleiter

## Patentansprüche

1. Endoskopisches System (1) mit in einer proximalen Versorgungseinheit (4) angeordneter Anregungsstrahlenquelle (5), mit einer optischen Strahlungs-Übertragungsstrecke in einem Einführungsteil (3) und einem distalseitigen Fluoreszenzumsetzer, wobei als Anregungsstrahlenquelle (5) eine im kurzwelligen sichtbaren Spektralbereich emittierende Laserdiode (6) und als optische Übertragungsstrecke eine Glasfaser (8) vorhanden sind und der Fluoreszenzumsetzer zur Umwandlung in Weißlicht geeignet ist, und bei dem als Fluoreszenzumsetzer ein Fluoreszenzkörper (12, 22) als separates, austauschbares Bauteil der Lichtaustrittsfläche der Glasfaser (8) nachgeordnet ist, **dadurch gekennzeichnet, daß** der Fluoreszenzkörper (12, 22) in einem an das Einführungsteil (3) ankoppelbaren Wechselkopf (16) angeordnet ist, der zur Erzeugung eines Beleuchtungs- und/oder Meßstrahlenbündels mit weiteren optischen und wärmeableitenden Bauelementen ausgebildet ist.

2. Endoskopisches System nach Anspruch 1, **dadurch gekennzeichnet, daß** am distalseitigen Ende des Einführungsteils (3) eine Abbildungslinse (20) zur Fokussierung des aus der Lichtaustrittfläche der Glasfaser (8) austretenden Anregungsstrahlenbündels auf den Fluoreszenzkörper (12, 22) angeordnet ist.

3. Endoskopisches System nach Anspruch 1, **dadurch gekennzeichnet, daß** dem Fluoreszenzkörper (12, 22) in dem Wechselkopf (16) ein Wärme ableitendes Fenster (15) vor- oder/und nachgeordnet ist.

4. Endoskopisches System nach Anspruch 1, **dadurch gekennzeichnet, daß** der Fluoreszenzkörper (12, 22) zwischen zwei Wärme leitende Fenster (15) eingepaßt ist.

5. Endoskopisches System nach Anspruch 3 oder 4, **dadurch gekennzeichnet, daß** mindestens ein Fenster (15) als Diamantscheibe oder transparentes, diamantbeschichtes Element ausgeführt ist, das auch optisch abbildende, reflektierende, streuende oder beugende Eigenschaften besitzen kann.

6. Endoskopisches System nach Anspruch 1, **dadurch gekennzeichnet, daß** am distalseitigen Ende des Einführungsteils (3) eine Abbildungsoptik (21) zur Erzeugung eines kollimierten Strahlenbündels aus dem aus der Lichtaustrittsfläche der Glasfaser (8) austretenden Anregungsstrahlenbündels angeordnet ist.

7. Endoskopisches System nach Anspruch 6, **dadurch gekennzeichnet, daß** in dem Wechselkopf (16) eine Abbildungslinse (20) zur Fokussierung des kollimierten Anregungsstrahlenbündels auf den Fluoreszenzkörper (12, 22) angeordnet ist.

8. Endoskopisches System nach Anspruch 6, **dadurch gekennzeichnet, daß** in dem Wechselkopf (16) ein optisches Element (14) zur Anpassung der Strahlungsdichte des kollimierten Anregungsstrahlenbündels an die geometrischen und fluoreszenzoptischen Eigenschaften des Fluoreszenzkörpers (12, 22) angeordnet ist.

9. Endoskopisches System nach Anspruch 1, **dadurch gekennzeichnet, daß** in dem Wechselkopf (16) lichteintrittsseitig ein Strahlen-Umlenkelement (17) angeordnet ist.

10. Endoskopisches System nach Anspruch 9, **dadurch**
**gekennzeichnet, daß** das Strahlen-Umlenkelement (17) als Strahlenteiler (23) ausgebildet ist.

11. Endoskopisches System nach Anspruch 10, **dadurch gekennzeichnet, daß** der Strahlenteiler (23) als Würfelelement mit strahlenteilender und strahlenumlenkender Kittfläche ausgebildet ist.

12. Endoskopisches System nach Anspruch 10, **dadurch gekennzeichnet, daß** in der Versorgungseinheit (4) mindestens eine weitere Strahlenquelle mit Emission im roten oder grünen Spektralbereich und zur Einkopplung in die Glasfaser (8) vorhanden ist, wobei der Strahlenteiler (23) mit einer dichroitischen Beschichtung zur Reflexion oder Transmission der weiteren Strahlung versehen ist.

13. Endoskopisches System nach Anspruch 1, **dadurch**
**gekennzeichnet, daß** dem Fluoreszenzkörper (12, 22) lichtaustrittsseitig optische Abbildungsmittel (14, 18) zur Erzeugung eines Beleuchtungsstrahlenbündels mit vorgegebener Apertur vorgeschaltet ist.

14. Endoskopisches System nach Anspruch 1, **dadurch**
**gekennzeichnet, daß** der Fluoreszenzkörper (12, 22, 25) ringsum von einem transparenten wärmeleitenden Medium (31) umgeben ist.
